**Europäisches Patentamt**

**European Patent Office**

**Office européen des brevets**

(11) Publication number : **0 467 695 A2**

# (12) EUROPEAN PATENT APPLICATION

(21) Application number : **91306579.3**

(22) Date of filing : **19.07.91**

(51) Int. Cl.⁵ : **A61N 1/362, A61N 1/39**

(30) Priority : **20.07.90 US 556549**

(43) Date of publication of application :
**22.01.92 Bulletin 92/04**

(84) Designated Contracting States :
**DE FR GB**

(71) Applicant : **TELECTRONICS N.V.**
**De Ruyterkade 58A P.O. Box 837**
**Curaçao (AN)**

(72) Inventor : **Daniel, Keith P.**
**59 St. John's Avenue**
**Gordon N.S.W. 2072 (AU)**

Inventor : **Collins, Kenneth A.**
**41 Shellcove Road**
**Neutral Bay, N.S.W. 2080 (AU)**
Inventor : **McCulloch, Roy M.**
**5 Elderberry Place**
**Cherrybrook, N.S.W. 2120 (AU)**
Inventor : **Wickham, John P.**
**20 Hampden Road**
**Five Dock, N.S.W. 2046 (AU)**
Inventor : **Gilli, Norma L.**
**20 Cooleena Road**
**Elanora Heights, N.S.W. 2101 (AU)**

(74) Representative : **Sturt, Clifford Mark et al**
**MARKS & CLERK 57-60 Lincoln's Inn Fields**
**London WC2A 3LS (GB)**

(54) **Detecting and treating myocardial infarctions in antitachy- arrhythmia and bradycardia pacing device.**

(57)    An apparatus and method are disclosed for treating a patient who is at risk of suffering a myocardial infarction (MI). Electrical activity of the patient's heart is sensed and signalled in order to detect the presence of an MI, and a thrombolytic drug is released into the bloodstream upon such detection. The apparatus and method optionally include provisions for sensing fibrillation, tachycardia and bradycardia of the heart and supplying corrective therapy in response thereto. Sensors may be employed to sense and signal mechanical activity of the heart, concentrations of selected ions in the patient's blood and selected serum enzyme in the blood, and the sensed signals used to supplement the sensed electrical activity signal in providing for recognition of the MI.

EP 0 467 695 A2

Fig.1.

PARALLEL PROCESSOR

MEMORY — 28

26

22

FILTER

18

14

38

40

42

36

CONTROLLING MICROPROCESSOR — 24

MEMORY — 25

EOL

THERAPY CONTROL UNIT

30

BATTERY

32

DEFIBRILLATION

44

BRADYCARDIA SUPPORT PACING

46

ANTITACHYCARDIA PACING

48

11

20

12

TELEMETRY AND WARNING — 34

57

DRUG STORAGE — 54

56

DRUG PROGRAM — 52

50

DRUG THERAPY

10

The present invention relates to a method and apparatus for detecting and treating myocardial infarctions in an implantable antitachyarrhythmia and/or bradycardia pacing device.

The term myocardial infarction (MI) refers to death of myocardial tissue resulting from an insufficiency of blood supply in either a relative or an absolute sense. This can occur in the form of micro-infarcts, or on a larger scale.

In the classical acute MI there is a sudden occlusion of a coronary artery due to thrombosis resulting in the death of part of either the right or left ventricular wall. The thrombus occurs due to atheromatous changes in the blood vessel wall. In a variant form, the infarct is due to spasm of a coronary artery producing sufficient narrowing of its lumen, and thereby blood carrying capacity, to cause damage.

In many cases death occurs within minutes of ischemic symptoms before any acute myocardial necrosis can be diagnosed or demonstrated. It has been found that in the United States alone, death due to MI occurs in approximately 500,000 persons per year. Furthermore, it has been found that a large number of people who have experienced MI's are at the risk of suffering from a further infarct.

It is now recognized that the process of coronary artery thrombosis can be reversed with agents that lyse the bloodclot. There are identifiable patients who may be saved from the danger of further infarction and/or sudden death by thrombolytic therapy, provided there is minimal delay (less than forty minutes) between coronary artery occlusion and the onset of therapy. However, it has been shown that some benefit may be derived from therapy applied up to six hours after the onset of the MI.

An MI is usually accompanied by chest pain (angina). This is the key symptom that drives a patient to seek medical help. In these cases the self-detection of a heart problem by the patient results in the patient being transported to a hospital or medical center as soon as possible for diagnosis and treatment. At times, due to the suddenness of the MI, the whereabouts of the patient, transportation difficulties, or other factors, there is not sufficient time available for treatment to be initiated. The patient's death or irreparable damage to his heart becomes unavoidable. At other times there is no chest pain, or chest pain may occur well after the actual occlusion, or the patient may be unable to respond to the pain due to unconsciousness.

Accordingly, there is a need for a device for detecting MI at onset by objective methods without the need for relying on the patient's chest pain as an instigator for causing the patient to proceed to a medical diagnosis center. This device must be able to warn the patient of his perilous state and to initiate therapy to minimize the effect of the infarct or to abort the infarct.

## MI DETECTION

Present methods of detecting MI's are based on clinical examination, electrocardiographic changes, enzymatic and metabolic changes, two-dimensional echocardiography, wall movement abnormalities, radionuclide imaging and scanning, magnetic resonance imaging, haemodynamic changes and the presence of fibrillation.

## Clinical Examination

The first indication of an MI is usually the particular symptoms experienced by the patient. These alone are not reliable indicators as they may be similar to those of patients having experienced prolonged or severe angina attacks (an eposide of ischemia), or intermediate coronary insufficiency syndrome.

Typically, the patient appears complaining of a crushing retrosternal chest pain that radiates up into the neck and down the arms. The patient is usually pallid and sweaty. On examination, there are no signs specific to an acute MI. The patient may be hypotensive or hypertensive, bradycardic or tachycardic. Arrhythmias are often present. Signs of heart failure are frequently seen.

## Electrocardiographic Changes

## ST Segment Changes

The ST segment is the portion of the ECG between the end of the QRS complex, also called the J point, and the commencement of the T wave.

During an MI, the earliest change seen in the surface ECG is inversion of the T wave. This occurs as early as 15 seconds after the interruption of coronary flow. This is followed quickly by ST segment elevation, and reversion to upright T waves (on the same side of the iso-electric line as the original T waves), with increased amplitude. The R-wave amplitude increases and the S wave amplitude decreases.

It has been found that the TQ-ST segment changes in the first few hours following an infarction may be due to progressive electrical and biochemical dysfunction of the cell membrane due to ischemia. In early ischemia (0-20 minutes), the changes may be due to the extracellular accumulation of potassium. The decrease in the magnitude of the TQ-ST segment deflection in the hours, or days, after acute MI, may be in part due to the loss of electronic coupling between the myocardial cells. ST segment elevation is indicative of epicardial injury and implies the presence of a zone of damaged myocardial tissue. The explanation for ST changes depends on the existence of a boundary between damaged and normal tissue.

Such a zone, with varying and abnormal electrical characteristics, results in abnormal current flows during both electrical systole and diastole. The injured region is either incompletely repolarized or in a state of complete depolarization. Experiments involving intracellular recordings have confirmed that incomplete repolarization exists in the area of injury during electrical diastole. It would appear that the ratios of intracellular to extracellular $K^+$ and $Na^+$ ions may affect these ST segment changes.

Q- and R-wave Changes

The Q-wave is the initial negative deflection of the QRS complex and represents ventricular depolarization. The appearance of a Q-wave represents the loss of electrically active myocardium.

The present inventors have demonstrated that an initial increase in R-wave amplitude occurs within five minutes from the onset of chest pain. Significant loss of the R-wave is detectable at thirty minutes, and is complete within six hours of the onset of chest pain. After twelve hours from the onset of chest pain, changes in the R/S ratio, and renewed appearance of Q-waves cannot be detected using precordial mapping.

While there may be a significant reduction in ST segment elevation in surface ECG's immediately after successful recanalization, it has also been found that new Q-waves and/or R-wave reduction often appear in recanalized patients.

It has also been observed, by means of precordial mapping, that the size of the infarct may be defined. Precordial mapping has also shown consistent changes in the ST segment, Q-wave development and the loss of R-wave amplitude.

Biochemical Changes

The result of an MI is the death and then autolysis of myocardial cells. The cellular contents are released into the interstitial fluid and diffuse into the blood stream. Larger molecules enter the blood stream more indirectly via the lymphatics. Cells that have suffered ischemic damage will also leak some of their cellular contents even though they may ultimately recover from the insult. Profound disturbances of membrane function occur with ischemia; especially in the function of the ionic poumps.

The presence of these cellular contents in the bloodstream can be determined by biochemical assay. Most of the contents of myocardial cells, e.g. $Mg^+$, $Na^+$ and $K^+$ ions, are present in other cells and in the blood stream at a steady concentration. However a sudden rise in their concentration is a key indicator of cellular damage. There are some molecules that are found principally in myocardial cells that can be used to specifically diagnose cellular damage; e.g. the so-called "cardiac enzymes" such as the creatine kinase MB fraction.

There are other biochemical markers of an MI. There are major metabolic responses and adaptations to an MI that produce detectable biochemical changes in the blood. An MI usually initiates a massive outpouring of catecholamines and is associated with major changes in the normal mechanisms of cellular energy exchange. Serum lactate, inorganic phosphates, pH, and free fatty acids are known as variants with MI's. However, of the biochemical changes used in the diagnosis of MI, the following have been shown in studies to be the most useful.

CPK-MB

Creatine kinase isoenzyme MB (CPK-MB) is found predominantly in the myocardial cell. It is one of a family of enzymes present, in one form or another, in all muscle cells. CPK-MB is released into the blood stream with damage to, or death of, myocardial cells.

There is normally little or no CPK-MB detectable in blood; there is normally a detectable level of other CK isoenzymes.

Other factors can cause an increase in serum CPK-MB levels. These could be an increase in serum CPK-MB due to either cardiac injury, other than acute myocardial infarction, or a decreased clearance of enzyme, or the presence of rare malignancies. It has been shown that if the CPK-MB/total CPK ratio of 80 ng/U was used for differentiating myocardial necrosis from muscular injury, no AMI would be missed and no trauma would be falsely treated. Therefore, it is the ratio of CPK to CPK-MB which is important in MI detection. In addition, massive increases in CPK-MB alone are also diagnostic of an AMI.

Success in reperfusion can be monitored by changes in the CPK level, because serum CPK-MB rapidly increases immediately after recanalization, reaching an early peak. Peak serum levels are reached in approximately fifteen hours in successfully recanalized patients and twenty six hours in the unsuccessful patients. After successful reperfusion, the levels rise within seconds of recanalization of the occluded coronary artery.

It has been found that serum CPK-MB does not increase after an electrical countershock. This is therefore not likely to impair detection of an evolving myocardial infarction.

It would appear that CPK-MB is valuable not only as an indicator of acute myocardial infarction, but also as an indicator that reperfusion has occurred.

Potassium Ions

$K^+$, $Na^+$, and $Ca^{++}$ ions are the major ions involved in the electrical conduction of the heart. $K^+$ is the predominant intracellular electrolyte, while $Na^+$ and $Cl^-$ are the predominant extracellular electrolytes.

One of the earliest events in ischemia is a rise in serum K⁺ level, with concomitant cell membrane depolarization. This occurs within seconds after occlusion. It appears that depolarization alters the excitability of the cells, thus setting the stage for the development of arrhythmias.

It has been found that, during the first minute following occlusion of arterial flow, conduction of the action potential into the ischemic region is accelerated and the current threshold for late diastolic threshold excitation is reduced. After that, conduction is reduced while the threshold current increases. It appears that this electrophysiological effect is due, in large parts, to a rise in extracellular K⁺ level linked with intracellular accumulation of Ca⁺⁺ ions. Thus, the increase in serum K⁺ level seen with an AMI can be used to assist in making the diagnosis of AMI.

Observations have shown that many episodes of VT/VF occur as a result of ischemia. Cellular calcium loading may affect the generation of arrhythmias when an MI or ischemia is present. The depolarization of myocardial cells in acute ischemia is due, in part, to excellular accumulation of potassium and intracellular accumulation of calcium.

As potassium is found in many other tissues and fluids within the body, it is necessary for sensors linking potassium changes with an infarction to differentiate those changes from those which occur due to other causes; e.g. diabetic ketoacidosis or other metabolic abnormalities. This can be done by using K⁺ changes in relationship to other sensed events, e.g. ST changes and/or a rise in CPK-MB, or by sensing K⁺ concentrations at two sites; one a large vein to reflect average whole body extracellular levels and one within the right ventricle to reflect any changes in blood being returned from the coronary circulation via the coronary sinus. Sensing changes in the concentration of K⁺ allows a more rapid response to an AMI. The use of the other sensors reduces the possibility of false positive activations of the device. It is preferable to include a lead in the SVC to compare with the other sensor values.

Wall Movement Abnormalities (WMA's)

Post MI, the infarcted section of the heart wall can be seen to move less (hypokinetic), stop moving (akinetic), or move in a disordered manner (dyskinetic) as compared to normal movement. Such abnormalities can be observed by most imaging systems used in cardiology, and have been extensively studied by conventional and digital angiography, radionucleotide imaging and echocardiographic means. These studies have shown that assessment of WMA's are an excellent indicator of an MI and are also useful for assessing the effect of thrombolytic therapy.

Studies of WMA's have shown that an infarct has a global effect on the mechanical movements of the heart, thereby enabling one sensor to be affected by an infarct in any position. Implantable sensors that can detect WMA are already available. A sensor system that can be used is an accelerometer placed on the tip of an intracardiac lead located on the septal wall. Alternatively, a sonomicrometric system can be used. Ultrasonic transducers are located in the tips of two screw-in leads placed on the ventricular wall and the separation between the two transducers is used as an indicator of the mechanical function of the heart.

Two-Dimensional Echocardiography

This procedure is used for determining the occurrence of WMA's after an MI. External transducers are used for the detection of increased left ventricular end diastolic volume and end systolic volume. The detection of WMA is performed by a transesophageal echocardiographic transducer enabling continuous monitoring during the evolution of MI's. The procedure is normally performed at the patient's bedside and has more specific application in the detection of a left ventricular mural thrombus.

Radionuclide Imaging

Radionuclide imaging involves the injection of Technetium-99m (TC-99m) pyrophosphate. The radioactive material is absorbed or adhered selectively to areas of recent myocardial necrosis and allows the application of imaging techniques for MI detection. Due to the limited resolution of simple planar images, the method is more useful for large infarcts that tend to be easily diagnosed by other methods. Single photon emission computed tomography has been used in association with the technique for revealing the size of the infarct.

Thallium-201 Scanning

Thallium-201 scanning reveals myocardial perfusion. The technique shows abnormal perfusion in patients with acute or old MI's. It has been shown to have about a sixty percent success rate in cases of proved MI. However, false negatives tend to appear with inferior infarcts and in ones with lower CK levels (peaks less than 3 times the upper limit of normal). Additionally, false positives are a frequent occurrence with the technique.

As with radionuclide imaging, the method requires large and expensive instrumentation for its application.

Magnetic Resonance Imaging

Magnetic resonance imaging is used for providing images of acute MI's. With ECG synchronization, infarcts are evident as areas of high signal intensity and prolonged relaxation times.

## Haemodynamic Changes

Myocardial ischemia is commonly associated with changes in blood pressure, being either elevation or depression. A detection method for MI has been the continuous monitoring of blood pressure. However, this method has been regarded as unreliable due to other factors, such as pain, which cause variations in blood pressure.

Another haemodynamic measurement that has been used in MI detection is pressure in the left atrium. Indirect measurements have been taken with a Swann-Ganz catheter measuring the pulmonary-capillary wedge pressure which is considered as being equal to the left atrial pressure. Variations in the left atrial pressure correspond to variations in the left ventricular end diastolic pressure which increases at the onset of an MI.

The left ventricular end diastolic pressure may also be measured via a catheter introduced into the aorta. Left atrial pressure may be measured directly in those individuals who have had to undergo open heart surgery by introducing a catheter into the left atrium at the time of surgery. This method is not regarded as highly reliable, as an MI is not always associated with an increase in left ventricular end diastolic pressure. This method is also limited by its invasive nature.

All of the above detection methods include externally operated devices or methods. This may require a visit by the patient to a hospital or medical center following the onset of an acute MI. Many patients appear for treatment only after irreversible damage has been done to the myocardium. In some cases, the severity of the MI causes patient death before detection and treatment in the hospital or medical center. The methods are all external, non-automatic, and temporary and may require large and expensive equipment. Further, when these methods are used singly, each has its own false negative and false positive diagnosis rate. Also, certain temporary invasive procedures may be undesirable and time-consuming and may not have high reliability when used as a per se MI detection method.

## Ventricular Tachycardia and Fibrillation

Ventricular tachyarrhythmias occur in patients with acute MI. VT is frequent in about ten percent and VF in about fifteen percent of patients. Ectopic beats are known to occur in the ventricle in over ninety percent of patients suffering an acute MI. Ventricular tachyarrhythmias may be the result of delayed conduction through damaged myocardium or due to enhanced automaticity (which involves an increased intrinsic discharge rate) of damaged ventricular cells, or may be due to the dispersion of refractory periods seen in damaged myocardium.

The association between acute MI's and ventricular tachyarrhythmias establishes the need for an implantable antitachyarrhythmia device which not only detects tachyarrhythmias and provides antitachyarrhythmia therapy, but also detects MI's and provides therapy for acute MI's.

## Thrombolytic Drug Therapy

Thrombolytic drug therapy is commonly used in treating acute MI's. The purpose of the therapy is to reopen the occluded artery and restore perfusion to the infarcted area with minimal delay so as to prevent or arrest myocardial damage.

Thrombolytic agents act by a common mechanism; they all activate plasminogen, which is the zymogen of plasmin. Plasmin is a serine protease that digests fibrin, the principal component of a blood clot.

Streptokinase is widely used as a thrombolytic agent, and acts indirectly by forming a streptokinase-plasminogen complex thereby converting plasminogen to plasmin. Its effectiveness has been proven in several large trials (e.g. G.I.S.S.I. "Effectiveness of Intravenous Thrombolytic Treatment in Acute Myocardial Infarction" Lancet I: 397-401 (1986). I.S.I.S.-2 "Randomized Trial of Intravenous Streptokinase, Oral Aspirin, Both or Neither Among 17,187 Cases of Suspected Myocardial Infarction: ISIS-2" Lancet II: 349-360 (1988))

Tissue plasminogen activator (TPA) has been shown to re-open occluded arteries in a high percentage of cases. This is a plasminogen activator which is found in human endothelium that is now produced via recombinant DNA technology. In vivo, it acts principally in the thrombus matrix and activates tissue-bound plasminogen. When infused, it will activate plasminogen in the presence of fibrin.

Anisoylated streptokinase plasminogen activator complex (APSAC) comprises a complex of a molecule of streptokinase linked to one of plasminogen with an anisoyl group covering the compound's active site. The compound is deacylated in vivo; the two portions are cleaved in the blood stream allowing the streptokinase to become active.

Single-chain urokinase plasminogen activator, or pro-urokinase, is a single-chain precursor of urokinase which has a strong affinity for plasminogen and activates plasminogen directly in the presence of fibrin.

There is a need for an implantable device which automatically applies thrombolytic drug therapy in response to detection of the onset of coronary artery thrombosis. The thrombolytic drug may be delivered separately or in combination with other drugs such as an antiarrhythmia agent or a beta-blocking drug. The determination depends on clinical efficacy, stability of the drugs during storage, whether the dose should be applied as a bolus or by continuous infusion, and the total volume of the drug and its solution.

It is preferable that the automatic MI detection and drug delivery device be in combination with an implantable pacer defibrillator/cardioverter. However, it may also be structured as an external system.

Other Drug Therapy

The use of both nitrates and beta blockade have been shown to be of benefit in the management of MI. Nitrates are the treatment of choice for reversing coronary artery spasm. The reduction in mortality after an MI due to the use of beta blockers is well known. There is the suggestion that early treatment with those agents may be of added benefit.

Antiarrhythmic drugs are frequently used in the treatment of the arrhythmias that occur during and after an acute MI.

Prior Art Patents

U.S. patent No. 4,750,494 to King entitled "Automatic Implantable Fibrillation Preventer" discloses sensor technology using a potassium sensor for prediction of ventricular fibrillation.

European Patent Application 87300496-4 by Goor describes an apparatus and method for detecting myocardial ischemia by monitoring systemic vascular resistance. Myocardial ischemia is considered to be present when the systemic vascular resistance increases by at least sixty percent. The apparatus comprises a flexible catheter tube inserted into the artery and a micro-manometer embedded in the outer face of the wall of the catheter. The outer face of the embedded micro-manometer is directly exposed to the blood in the artery. The technique monitors the systemic vascular resistance as described in U.S. Patent No. 4,429,701 to Goor.

U.S. Patent No. 4,146,029 entitled "Self-Powered Implanted Programmable Medication System and Method" to Ellinwood discloses an implanted medication system with some external control. In this particular system, a sensor which may be either internal or external is arranged on the body to sense some kind of physiological, chemical, electrical or other condition at a particular site in the body, thereby providing data corresponding to the sensed condition at the sensed site. The data is then channeled to an implanted, microprocessor controlled medication dispensing device. A predetermined amount of medication is then dispensed in response to the sensed condition according to a pre-programmed algorithm in the microprocessor control unit.

The system also has an extra-corporeal coding pulse transmitter for selecting between different algorithms in the microprocessor control unit. However, the device does not disclose any sensor capable of detecting MI's. Furthermore, the system does not disclose any method of treatment of MI's, i.e. thrombolytic drug therapy. In the management of AMI, the system is limited to overdrive pacing and antiarrhythmic agents on a temporary basis with the assumption that an MI is merely a "transient" condition. Furthermore, the device is unable to provide defibrillation therapy to revert the ventricular tachyarrhythmias associated with MI.

In addition, in U.S. Patent No. 4,146,029 the algorithms which control the rate of infusion are programmed into the implanted unit. Thus, the algorithms in the device cannot be upgraded without surgery. The extra-corporeal controller in the system is limited to selecting a particular one of several medication programs. A problem therefore exists in that the programs in the device cannot be altered.

Terminology

As used herein, antitachycardia pacing will mean any pacing for the reversion of tachycardia. Tachycardia refers to any fast abnormal rhythm of the heart which may be amenable to electrical discharges and specifically includes ventricular tachycardia (VT), supraventricular tachycardia (SVT), ventricular flutter and/or ventricular fibrillation (VF) and may include atrial fibrillation or flutter.

The term therapy as used herein includes the processes used between the detection and reversion of a tachyarrhythmia and includes the actions of antitachycardia pacing, cardioversion and/or defibrillation shocks. The term cardioversion refers to the discharge of electrical energy into the cardiac tissue in an attempt to terminate or revert a tachyarrhythmia. This may take the form of a high energy discharge (up to 40 Joules of more) or a low energy discharge (less than 1 Joule). Cardioversion shocks may or may not be synchronized to the rhythm of the heart. Defibrillation is a particular example of cardioversion.

This invention applies equally to devices which deliver energy synchronized to an R-wave and to those that do not, and applies to devices which use lower energy pulses (up to 1 Joule) as well as to devices which use higher energy pulses (up to 40 Joules or more). The invention applies to devices which deliver cardioverting shocks alone as well as to devices which deliver antitachycardia pacing pulses alone or in combination with cardioverting shocks. The invention will usually apply to ventricular implantable cardioverters, but is equally applicable to atrial cardioverters or multiple chamber cardioverters or defibrillators. The invention applies also to the delivery of any antitachycardia pacing pulse and post-reversion pacing therapy.

Templating refers to a technique for storing a reference segment of signal and then using this segment to find subsequent changes in later signals obtained from the same source.

It is an object of the invention to provide a device capable of achieving a significant reduction in mortality due to coronary artery disease by the early detection, diagnosis and treatment of MI's.

It is a further object of the invention to provide a device which reduces the incidence of death due to ventricular tachyarrhythmias and congestive heart failure.

It is a further object of the invention to provide a greater reduction in patient mortality as a result of MI or associated ventricular tachyarrhythmias over that obtained with existing diagnoses and therapies.

It is a further object of the invention to provide a device for detecting MI at onset by objective methods without the need for relying on a patient's chest pain as an instigator for proceeding to a medical diagnosis.

It is a further object of the invention to provide an implantable automatic device for the detection of MI.

It is a further object of the invention to provide an implantable automatic device for the detection and the treatment of MI.

It is a further object of the invention to provide an implantable automatic device for the detection and the treatment of MI in combination with a defibrillator and pacing device.

It is a further object of the invention to provide a device which detects MI's and provides the appropriate therapy with minimal delay between the onset of an MI and the application of the therapy.

It is a further object of the invention to provide an implantable device which automatically detects and diagnoses MI's and provides appropriate therapy by means of a drug storage, loading, and pump system.

It is a further object of the invention to provide a programmable medical device which automatically detects and diagnoses MI's, provides either the appropriate thrombolytic therapy or monitors the need to apply such therapy, and which delivers electrical energy to cardiac tissue in an attempt to revert tachyarrhythmias and restore normal sinus rhythm and provides bradycardia therapy should it be required.

According to the invention, there is provided an implantable medical device comprising means for detecting the presence of a myocardial infarction. The device may include means for alerting the patient when an MI is detected, and means for applying MI therapy to treat the patient.

There is further provided an implantable medical device comprising means for detecting tachyarrhythmias and means for providing antitachyarrhythmia therapy along with means for detecting the presence of an MI, means for applying MI therapy and means for alerting the patient of a detected MI.

Preferably, the MI detecting means includes at least two sensing systems. Preferably it includes a series of sensing systems which comprise an electrocardiographic sensor, a mechanical sensor, a serum enzymatic sensor, and a serum ion/metabolite sensor.

The electrocardiographic sensor preferably measures ST segment changes, Q-wave changes or R-wave amplitude changes, or a combination of these.

The serum enzymatic sensor preferably measures changes in CPK-MB.

The ionic sensor preferably measures changes in potassium levels.

The device also includes means for storing a drug along with an infusion means for dispensing the drug from its storage to the patient in response to the detection of an MI condition. The drug may be one or a combination of thrombolytic drugs and may also include one or more antiarrhythmic agents and/or nitrates and/or other cardiovascular drugs.

Preferably the device includes means for monitoring the reversion of an MI following application of MI therapy so that therapy is stopped at a successful reversion. Electrocardiographic changes are monitored by a combination of ST segment changes, Q-wave changes, and R-wave amplitude changes.

The device is preferably externally programmable to vary detection and therapy application criteria. For example with respect to a serum enzymatic sensor, there may be a programmable value for the CPK-MB/total to CPK ratio that is alterable to reflect alterations in this ratio due to chronic myocardial ischemia. Preferably, the apparatus has the capacity to be programmed to automatically adapt to alterations in the baseline values and morphology (templating) of the sensed parameters. For example, programmable potassium levels may be provided for the ionic sensor to vary the base line criteria. Similarly, the response of the device to the patient's electrical rhythms may be programmable including the ability to activate an automatic learning mode. Electrocardiographic values may be regularly measured to establish a baseline for a particular patient.

The invention also provides a method of treating MI in an implantable medical device comprising the steps of detecting the presence of an MI; alerting the patient to the presence of an MI; and applying MI therapy to treat the MI.

Additionally, the invention provides a method of treating MI in an implantable antitachyarrhythmia device comprising the steps of detecting the presence of an MI; alerting the patient to the detected MI; applying MI therapy to treat the detected MI; detecting the presence of a tachyarrhythmia; and applying antitachyarrhythmia therapy to treat a detected tachyarrhythmia.

Preferably the method also includes the steps of detecting a bradycardia condition and applying bradycardia pacing therapy in response to a detected bradycardia condition.

Further objects, features and advantages of the invention will become apparent upon consideration of the following detailed description in conjunction with the drawings, in which:

FIG. 1 is a block diagram of an apparatus for detecting and treating MI in an antitachyarrhythmia and bradycardia pacing device in accordance with the invention;

FIG. 2 is a flowchart for the detection of MI in an implantable device in accordance with FIG. 1;

FIG. 3 is a block diagram of an external apparatus for detecting and treating MI including antitachycardia and bradycardia supports features; and

FIG. 4 illustrates detection of an acute MI in a LAD animal model.

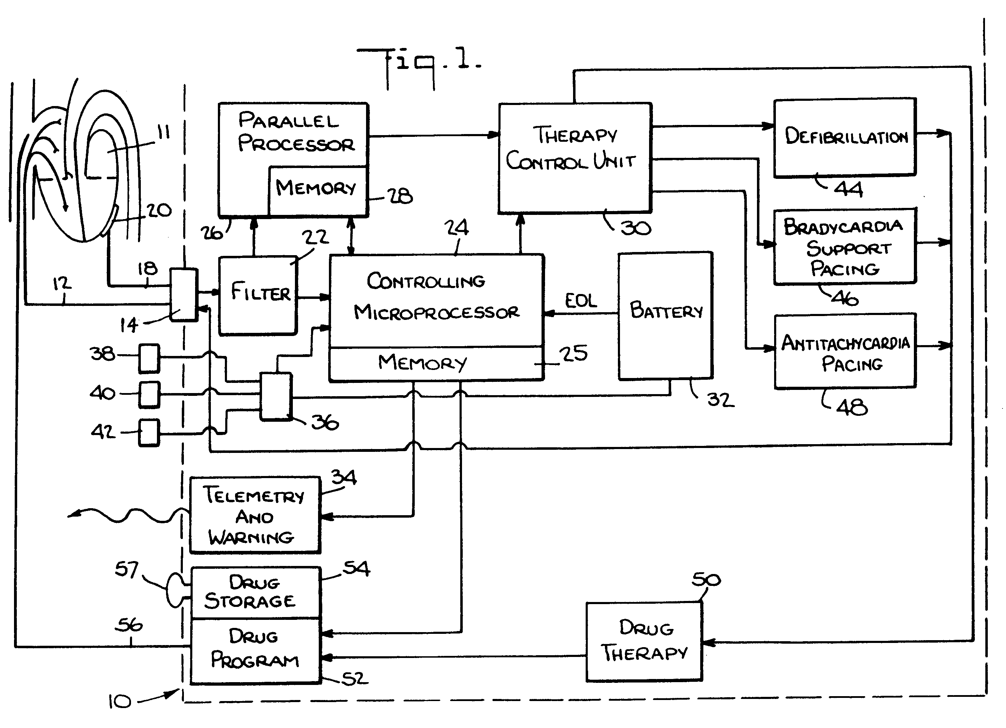

Referring to FIG. 1, an implantable apparatus 10 for treating and detecting myocardial infarction (including antitachycardia and bradycardia pacing circuits) is connected to the heart 11. A first lead 12, which may have its electrode positioned in the atrium, superior vena cava ventricle or on the cardiac surface, is connected to a suitable electronic switch 14 within apparatus 10. In the illustrated embodiment lead 12 advantageously extends into the right ventricle, where it may be used for sensing and pacing.

Another lead 18 which terminates in a cardiac surface patch 20 is also connected to switch 14, thus providing cardiac surface traces and a preferred electrode for defibrillation. In addition to the existing leads (12 and 18), there may be added up to six or more other sensing leads in order to reduce "false negative" readings and increase the efficiency of the device's ability to sense.

An example may include three additional sensing leads which are "massless" in the sense of having a small area sensing tip of Teflon coated wire. These would be subcutaneous leads that are tunnelled in at the time of implant. Two electrodes would be placed at the front of the chest with one being located over the fourth or fifth intercostal space, and the other being located over the fifth or sixth intercostal space; the third electrode would be placed at the patient's back over the seventh or ninth intercostal space, on either the right or left side.

Switch 14 is bi-directional in that signals from the heart carried by leads 12 and 18 are brought into apparatus 10 while electrical therapy for the heart 11 from apparatus 10 is routed through switch 14 to the heart 11, as more fully described below.

Signals from the heart 11 which travel through switch 14 are filtered in a filter 22. The output of filter 22 is provided to the digitizing input of a microprocessor 24 and a separate parallel processor 26. Parallel processor 26 may contain a memory 28 in which processing of signals recovered from the heart may occur under the control of microprocessor 24. Processing in parallel processor 26 may occur in a manner similar to the processing performed by neural networks. In other words various weights may be assigned to different inputs. Further, these weights may be changed by reprogramming from time to time as necessary to adjust to changing physiological conditions of the patient.

Microprocessor 24 contains its own memory 25 which includes a ROM (not shown) for storing a program to operate microprocessor 24 and a RAM (not shown) for temporary and computational storage. Parallel processor 26 and microprocessor 24 provide inputs to a therapy control unit 30 which selects appropriate therapy for the patient as described in more detail below.

Apparatus 10 is powered by a battery 32. Microprocessor 24 monitors the voltage of battery 32 and when it is below a predetermined level microprocessor 24 provides an end of life (EOL) battery signal to a telemetry and warning block 34 so that the patient may be made aware of this condition of the battery. Block 34 is bidirectional, i.e., the patient may wear a receiver (not shown) which responds to warning signals from block 34. In addition, block 34 may receive signals from a programmer operated by a physician to reprogram apparatus 10 in any of its functions, including thresholds and weighting factors for the detection of MI.

Battery 32 also provides power for a sensor control block 36 which provides processing capability for signals from up to three different types of sensors. These sensor types include a mechanical sensor 38, an ionic sensor 40 and a serum enzymatic sensor 42 for sensing the conditions indicative of myocardial infarction, as discussed above. A signal output lead from block 36 is provided as an input to microprocessor 24, which processes the signals from sensors 38, 40 and 42. Thus, microprocessor 24 provides additional inputs to therapy control unit 30 so that the appropriate therapy may be selected not only on the basis of electrical signals from the heart but also on the basis of signals from transducers 38, 40 and 42.

Therapy control unit 30 can select defibrillation therapy provided by a defibrillation block 44, bradycardia support pacing provided by pacing block 46, antitachycardia pacing provided by antitachycardia block 48 or drug therapy initiated by drug therapy controller 50. In appropriate circumstances, a combination of these therapies may be indicated. The determination of which therapy or therapies to initiate is made by the logic arrangement of therapy control unit 30 based on inputs from parallel processor 26 and microprocessor 24. For example, electrocardiographic inputs and signals from sensors 38, 40 and 42 may indicate that a drug should be administered. However, microprocessor 24 may be programmed to store in memory 25 appropriate flag values which indicate that a near toxic dose of a drug has been administered in the recent past. Thus, microprocessor 24 may inhibit the administration of additional quantities of the drug until a predetermined period of time has elapsed.

Optionally, a drug therapy controller 50 controls a drug pump 52 which pumps drugs from a drug storage chamber 54 into the bloodstream through a catheter 56, the distal end of which is preferably positioned in the bloodstream so that the drugs being pumped are rapidly and efficiently conveyed to the heart. A drug pump interface connector 57 serves as a loading port for the drug which is placed in apparatus 10 just prior to implantation.

When a condition indicative of immediate danger to the patient, such as myocardial infarction, is indicated, telemetry and warning block 34 may also supply a warning to the patient. This may be done by way of the receiver mentioned above, or by providing a low level muscle activiting shock to the patient.

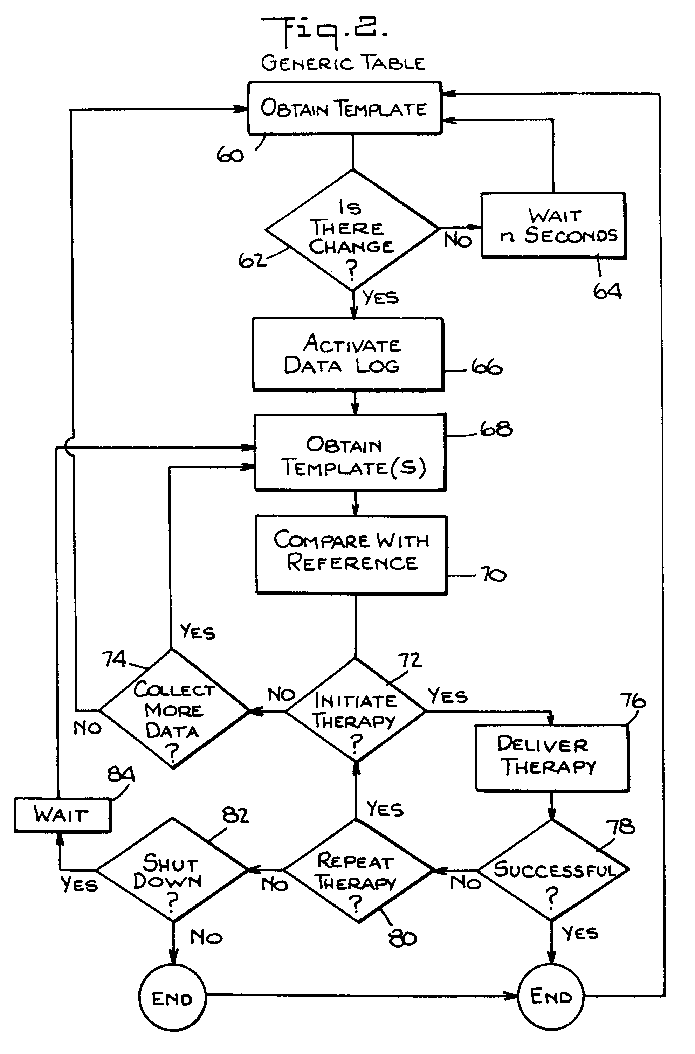

Referring to FIG. 2, a generic flowchart for the detection of myocardial infarctions is illustrated. At step 60 a baseline template for the patient is obtained of each of the sensor signals being used to monitor the condition. These may be the electrical signals from the heart, or the outputs of any one or more of sensors 38, 40 and 42 (FIG. 1). At step 62, a determination is constantly made as to whether there has been any change in the signal being monitored of sufficient magnitude to indicate the possibility of the commencement of a myocardial infarction. If there has been no change, then branching occurs to step 64 where the program waits for a predetermined period of time (N seconds) and branching back to step 60 occurs.

If there has been a change which indicates a possible myocardial infarction, then branching occurs to step 66 where a data log is activated. The data is stored in memory 28 of parallel processor 26 (FIG. 1). This data log produces another template at step 68. At step 70 a comparison of the template obtained at step 68 with the reference template (step 60) is made. If the comparison does not indicate a sufficient deviation in the nature of the template to warrant delivering therapy, branching occurs from step 72 to step 74. If the comparison at step 70 indicated minimal deviation, then a determination is made at step 74 to obtain no additional data and branching occurs back to step 60, where the original template is accessed. Monitoring continues as described above with respect to step 62.

If there is some deviation in the template when the comparison is made at step 70, but not enough to indicate that therapy should be initiated, then branching occurs from step 74 to step 68 where additional data is obtained as a result of continued data logging. If the comparison at step 70 then indicates that a sufficient deviation in the template has occurred to warrant the delivery of therapy, branching from step 72 to step 76 occurs where therapy is delivered.

At a predetermined time after the delivery of therapy, a determination is made at step 78, as to whether the therapy has been successful in terminating the infarction. If the answer is Yes, then operation is terminated and the system branches back to step 60 where the stored template is obtained. However, if the answer is No, then branching occurs to step 80 where a determination is made as to whether to repeat the therapy. If the answer to this inquiry is Yes, then branching occurs to step 72 and therapy is again delivered at step 76. If therapy has occurred for a maximum number of indicated times, such as a thrombolytic drug having been dispensed in sufficient quantity to become nearly toxic, then the determination made at step 80 causes branching to step 82 where a decision is made as to whether or not to shut down the unit for purposes of delivering additional therapy for the infarct. If the answer is No, then branching occurs back to step 60 where the original template is obtained. However, if the answer is Yes such as when a toxic level of a drug has been administered, then branching to step 84 occurs where a predetermined wait period is initiated. This wait period may be up to several hours and will be, in the case of the administration of a drug, a period of time at least sufficient to allow the level of the drug in the bloodstream to drop below near toxic limits. After the wait at step 84, branching to step 68 occurs where a current template is obtained based on data logging.

The flowchart of FIG. 2 contemplates that the apparatus according to the invention can "learn a state" so as to adjust the baseline values to which a comparison is made to determine whether a myocardial infarction has taken place. There are several ways in which this can be done. However, the preferable manner of performing this function is to obtain a template at step 60 which is representative of the current values of the parameter being monitored and to update this template periodically. Thus, the baseline value is adjusted at step 60, but the comparison with a reference at step 70 is responsive to any reasonably rapid change in the parameters being monitored.

In accordance with this concept, the baseline level of CPK-MB/total CPK ratio can be automatically adjusted. Further, the baseline level of the ion concentration being monitored, such as k+ may be adjusted automatically in this manner. The mechanical sensor 38, which may be used to monitor wall movements or blood pressure also provides inputs to apparatus 10 to be processed in the manner represented by the flowchart of FIG. 2 so that the baseline of comparison is constantly adjusted.

Baseline values may be adjusted when monitoring for Q-wave changes, R-wave amplitude changes or changes in the ST segment of the electrogram or electrocardiogram. For example, the ST segment is periodically sampled and always compared to the previous measurement.

In order to sample the R-wave it is necessary to change the characteristics of filter 22 (FIG. 1) when MI is present. The following criteria is used:

1. X out of Y of the previous cardiac cycle intervals are less than a predetermined length; and

2. R-wave amplitude has increased by at least 50 percent for a minimum of 10 minutes.

When these conditions have been met, the characteristics of filter 22 are changed so that it has a pass band between 0.5 and 5Hz. A template of the R-wave is made and the condition is monitored continuously. An alarm is then sounded if the following conditions are present:

A. X out of Y intervals are not shorter than a predetermined length;

B. The ST segment continues to change away from the normal; and

C. The R-wave amplitude decreases.

During the time that the characteristics of filter 22 is changed to have a pass band of between 0.5 and 5Hz, if the X out of Y criteria for tachycardia is present, the sample is saved and the filter is returned to the standard 50 to 60Hz pass band. The system then goes into a VT/VF detection mode and, if necessary, appropriate antitachyarrhythmia therapy is supplied. If a shock has been delivered, and the arrhythmia has been cleared, then monitoring with filter 22 in the 50 to 60Hz pass band mode continues for 2 minutes. The system then monitors appropriately as described above.

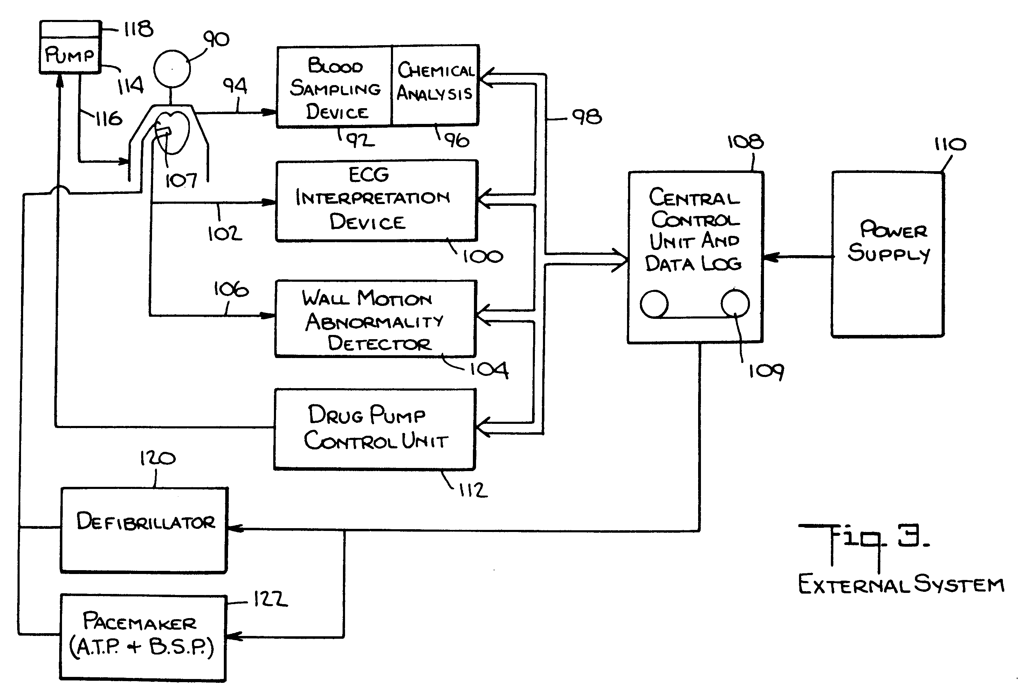

FIG. 3 illustrates an external system (as opposed to the implanted system of FIG. 1) in accordance with the invention. A patient 90 is connected to a blood sampling device 92 by means of an appropriate catheter 94. The blood taken from a patient is then chemically analyzed in a chemical analyzer 96. Data concerning its composition is placed on a data bus 98.

The patient is connected to an ECG interpretation block 100 by means of ECG leads 102. The ECG signals are processed and digital data representative of the ECG is also placed on bus 98 at the appropriate time by block 100.

The patient is also connected to a wall motion abnormality detector 104 by electrical leads 106 to a motion transducer 107 of a type described above. Digital data representative of the wall motion is placed on bus 98 at an appropriate time. Data from chemical analyzer 96, ECG interpretation block 100, and wall motion abnormality detector 104 are provided to a central control unit and data logger 108 by bus 98. Patient data may be continuously recorded on a recording medium such as magnetic tape 109 or in a large computer memory (not shown) in data logger 108. The receipt of appropriate data by data logger 108, which is powered by a power supply 110, activates an appropriate therapy delivery device. The therapy delivery devices include a drug pump control 112 which controls a pump 114 to provide predetermined doses of medication to the patient 90 through an intravenous tube 116. Quantities of the drug or drugs to be dispensed are stored in a reservoir 118 associated with pump 114.

Unit 108 can also activiate a control line to a defibrillator 120 and to a a pacemaker 122. Pacemaker 122 is preferably of the type which provides antitachycardia pacing and bradycardia support pacing. Defibrillator 120 may be any of several types well known in the art. For this external system, transthoracic defibrillation may be performed.

The operation of the system of FIG. 3 is similar to that of FIG. 1. The chemical analyzer 96 may include ionic and serum enzymatic sensors similar to those used in the system of FIG. 1.

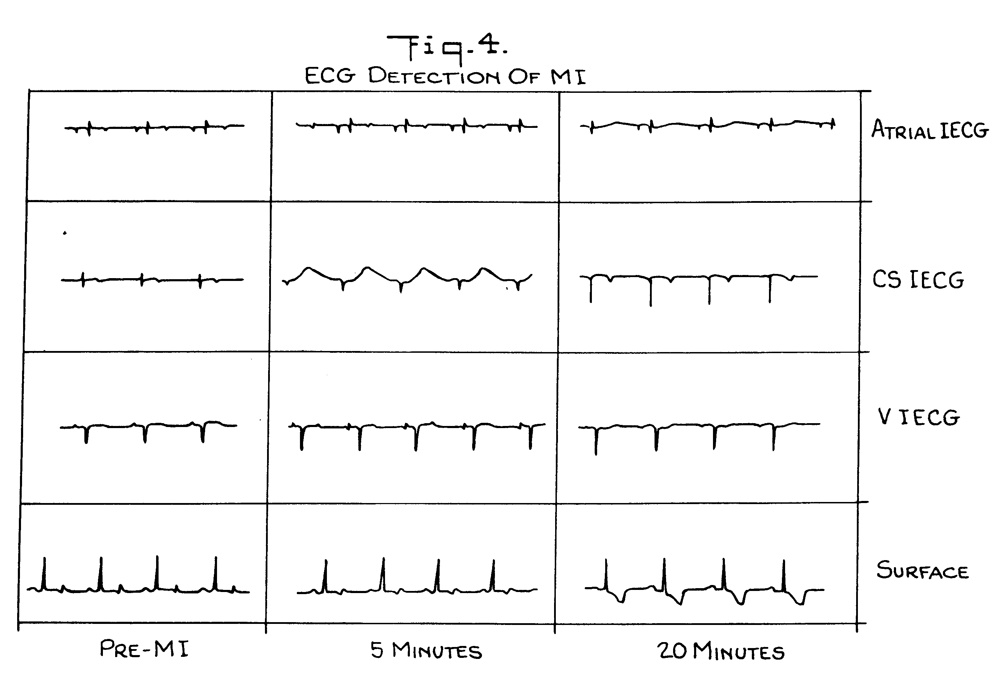

Referring to Fig 4, ECG detection of an MI as demonstrated in an LAD model in animals is illustrated. the traces illustrated therein are arranged in a matrix wherein the columns, from left to right, represent the pre-MI condition, the condition 5 minutes after the commencement of an MI, and the condition 20 minutes after the commencement of an MI. The rows, from top to bottom, represent the atrial IECG or electrogram, the cardiac surface electrogram, the ventricular electrogram, and finally the surface ECG.

It will be noted that there are virtually no changes in the atrial electrogram. However, there are profound changes in the cardiac surface electrogram from the pre-MI condition to the condition at 5 minutes. There is an amplitude decrease and there are changes in the ST segments of the electrogram. On the other hand, the ventricular electrogram and the surface ECG remain esentially unchanged at the 5 minute point. However, at the 20 minute point the R-wave of the cardiac surface electrogram shows increased amplitude and ST depression is present as well as a change in morphology. At 20 minutes, some amplitudes change as well as ST depression is noted in the ventricular electrogram. The surface ECG also exhibits marked ST depression at the 20 minute point.

This invention applies to antitachyarrhythmia devices capable of delivering one or more cardioversion or defibrillation shocks as well as to devices which deliver antitachycardia pacing pulses alone or in combination with cardioversion or defibrillation shocks. The antitachycardia pacing may be delivered to the atrium, the ventricle, or to both the atrium and the ventricle.

Although the invention has been described with reference to particular embodiments, it is to be understood that these embodiments are merely illustrative of the application of the principles of the invention. Numerous modifications may be made therein and other arrangements may be devised without departing from the spirit and scope of the invention.

## Claims

1. An apparatus for treating a patient who is at risk of suffering a myocardial infarction comprising:
   means for sensing electrical activity of the patient's heart;
   means responsive to electrical activity sensed by said sensing means for recognizing a myocardial infarction; and
   means for supplying a thrombolytic drug to the patient when said recognition means recognizes a myocardial infarction.

2. The apparatus of claim 1, further comprising:
   means for sensing fibrillation of the heart; and
   means for supplying defibrillation therapy to the heart when said fibrillation sensing means senses fibrillation.

3. The apparatus of claim 1, where said recognition means further recognizes a condition of tachycardia, further comprising means for supplying antitachycardia pacing pulses to the heart when said recognition means recognizes a tachycardia condition.

4. The apparatus of claim 1, wherein said recognition means further recognizes a condition of bradycardia, further comprising bradycardia support pacing means for pacing the heart when said recognition means recognizes a bradycardia condition.

5. The apparatus of claim 1, further comprising at least one of:
   a mechanical sensor responsive to mechanical activity of the heart;
   an ionic sensor responsive to concentration of a selected ion in the patient's blood;
   a serum enzymatic sensor responsive to a selected serum enzyme in the blood; and
   sensor signal processing means for processing signals from said at least one sensor to provide an input to said recognition means to supplement said sensed electrical activity and thereby aid said recognition means to recognize a myocardial infarction.

6. An apparatus for treating a patient suffering from heart disease comprising:
   means for sensing electrical activity of the heart,
   means for storing a representation of a selected portion of the sensed electrical activity indicative of baseline heart function,
   means for comparing subsequent sensed electrical activity with said representation of baseline heart function; and
   means for selecting a therapy means for the heart from a plurality of available therapy means based upon the manner in which the subsequent sensed electrical activity differs from said representation of baseline heart function; said plurality of therapy means including:
   i) means for supplying defibrillation shocks;
   ii) pacing means for supplying antitachycardia pacing pulses;
   iii) bradycardia support pacing means; and
   iv) means for supplying a drug to the patient.

7. The apparatus of claim 6, wherein said electrical activity sensing means includes at least one lead for receiving an electrogram from the heart.

8. The apparatus of claim 6, including housing means surrounding said apparatus, said housing means being for implantation.

9. The apparatus of claim 6, wherein said drug therapy means includes a drug storage means for storing drugs to be supplied to the heart.

10. The apparatus of claim 9, wherein said drug storage means stores at least one of a thrombolytic drug and an antiarrhythmic agent.

11. The apparatus of claim 6, further comprising at least one of:
    a) means for sensing mechanical activity of the heart; and
    b) means for sensing biochemical changes indicative of a change in the condition of the heart;
    wherein said storing means also stores data representative of at least one of mechanical and biochemical baseline function of the heart;
    said comparing means compares data representative of at least one of subsequent mechanical and biochemical function with respective stored data; and
    said therapy selection means also is responsive to comparisons of said stored and subsequent data representative of at least one of mechanical and biochemical function.

12. An Apparatus for detecting the presence of a myocardial infarction comprising:
    a) means for sensing electrical activity of the heart;
    b) a mechanical sensor means for sensing heart function and for providing a signal indicative thereof;
    c) a biochemical sensor means for sensing a change in blood chemistry associated with a myocardial infarction and for providing a signal indicative thereof;

d) means responsive to said electrogram and said signals for recognizing a myocardial infarction; and

e) means for alerting the patient when said recognizing means recognizes a myocardial infarction.

13. The apparatus of claim 11 or 12, wherein said mechanical sensor means comprises at least one of a heart wall motion detector and a blood pressure sensor.

14. The apparatus of claims 11 or 12, wherein said biochemical sensor means includes at least one of an ionic sensor and a serum enzymatic sensor.

15. The apparatus of claim 14, wherein said ionic sensor responds to potassium ions.

16. The apparatus for claim 14, wherein said serum enzymatic sensor responds to changes in CPK-MB level.

17. The apparatus of claim 12, further comprising means for applying therapy for treating a myocardial infarction when said detecting means detects a myocardial infarction.

18. The apparatus of any one of claims 1, 6 or 12 wherein said means for sensing electrical activity of the heart is responsive to changes in at least one of R-wave amplitude and ST segment voltage level.

19. A method for automatically monitoring the heart condition of a patient and administering a drug to treat myocardial infarction of the patient's heart comprising the steps of:

detecting the presence of myocardial infarction; and

providing therapy to the heart automatically to minimize the effect of said myocardial infarction; said therapy including releasing a thrombolytic drug into the bloodstream.

20. The method of claim 19, further comprising: detecting the presence of fibrillation; and automatically providing defibrillation therapy to the heart.

21. The method of step 19, further comprising detecting the presence of tachycardia; and providing antitachycardia pacing to the heart automatically when tachycardia is detected.

22. The method of step 20, further comprising detecting bradycardia, and providing bradycardia support pacing to the heart when bradycardia is present.

23. The method of claim 19, wherein said step of detecting myocardial infarction comprises at least one of the following steps:

detecting changes in electrical activity of the heart;

detecting changes in mechanical activity of the heart;

detecting changes in concentration of an ion in the patient's blood; and

detecting changes in concentration of serum enzymes in the blood.

24. The method of claim 19 wherein said step of detecting myocardial infarction comprises:

detecting changes in the electrical activity of the heart including at least one of change in R-wave amplitude and change in ST segment voltage level.

EP 0 467 695 A2

Fig. 1.

Defibrillation 44

Bradycardia Support Pacing 46

Antitachycardia Pacing 48

Therapy Control Unit 30

Battery 32

Controlling Microprocessor 24

Memory 25

EOL

Parallel Processor 26

Memory 28

Filter 22

Drug Therapy 50

Telemetry And Warning 34

Drug Storage 54

Drug Program 52

36

38

40

42

18

14

11

20

12

57

56

10

14

# Fig.2.
## GENERIC TABLE

Fig. 3.

Power Supply — 110

Central Control Unit And Data Log — 108 / 109

Blood Sampling Device — 92
Chemical Analysis — 96
ECG Interpretation Device — 100
Wall Motion Abnormality Detector — 104
Drug Pump Control Unit — 112

98
90 — 94 — 107 — 102 — 106

Pump — 114 / 116 / 118

Defibrillator — 120
Pacemaker (A.T.P. + B.S.P.) — 122

External System

16

# Fig.4.
## ECG Detection Of MI

| | Pre-MI | 5 Minutes | 20 Minutes | |
|---|---|---|---|---|
| | | | | Atrial IECG |
| | | | | CS IECG |
| | | | | V IECG |
| | | | | Surface |